(19) Europäisches Patentamt
European Patent Office
Office européen des brevets

(11) Veröffentlichungsnummer : **0 426 610 B1**

(12) **EUROPÄISCHE PATENTSCHRIFT**

(45) Veröffentlichungstag der Patentschrift :
**27.07.94 Patentblatt 94/30**

(51) Int. Cl.$^5$ : **C07C 237/46,** A61K 49/04

(21) Anmeldenummer : **90730014.9**

(22) Anmeldetag : **27.10.90**

# (54) Nichtionisches Röntgenkontrastmittel mit hohem Jodgehalt.

(30) Priorität : **03.11.89 DE 3937118**

(43) Veröffentlichungstag der Anmeldung :
**08.05.91 Patentblatt 91/19**

(45) Bekanntmachung des Hinweises auf die Patenterteilung :
**27.07.94 Patentblatt 94/30**

(84) Benannte Vertragsstaaten :
**AT BE CH DE DK ES FR GB GR IT LI LU NL SE**

(56) Entgegenhaltungen :
**EP-A- 0 015 867**
**FR-A- 2 293 919**

(73) Patentinhaber : **SCHERING AKTIENGESELLSCHAFT**
**D-13342 Berlin (DE)**

(72) Erfinder : **Speck, Ulrich, Dr.**
**Benediktinerstrasse 50**
**W-1000 Berlin 28 (DE)**
Erfinder : **Blaszkiewicz, Peter, Dr.**
**Rothenburgstrasse 25**
**W-1000 Berlin 41 (DE)**

EP 0 426 610 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft den in den Patentansprüchen gekennzeichneten Gegenstand, das heißt ein trijodiertes Isophthalsäurediamid, Verfahren zu dessen Herstellung, dieses enthaltende Röntgenkontrastmittel sowie dessen Verwendung zur Herstellung von Röntgenkontrastmittel.

Voraussetzungen für eine gezielte und erfolgreiche Therapie ist eine exakte Diagnose. Gerade auf dem diagnostischen Gebiet haben die Möglichkeiten in den vergangenen Jahren sehr stark zugenommen, wobei die Röntgendiagnostik in der Lage ist, praktisch jedes anatomische Detail selektiv und mit großer Genauigkeit darzustellen. In vielen Fällen werden die entsprechenden Strukturen aber erst durch die Anwendung von Röntgenkontrastmitteln sichtbar. Dies gilt insbesondere für die Blutgefäße, wobei die Entwicklung feinster Katheter und eine Weiterentwicklung der Röntgentechnik ebenfalls eine große Rolle spielen. Aus der bloßen diagnostischen Darstellung der Blutgefäße mittels Kathetern und Röntgenkontrastmitteln haben sich inzwischen eine Vielzahl von wenig-invasiven, kostengünstigen Behandlungsmethoden entwickelt. So ist es beispielsweise möglich, selbst Koronararterien unmittelbar nach der Darstellung einer Verengung im Röntgenbild mit Hilfe spezieller Katheter durch "Aufblasen" eines Ballons so aufzuweiten, daß ein annähernd normaler Blutfuß wieder hergestellt wird. Durch wiederholte Injektion von Kontrastmittel kann die genaue Lage des Ballons und die Aufweitung der Arterie Schritt für Schritt verfolgt werden. Auf diese Weise kann dem Patienten eine sehr aufwendige und belastende Operation am offenen Herzen erspart bleiben. Eine weitere therapeutische Anwendung in Verbindung mit der Gefäßdarstellung durch Katheter und Kontrastmittel ist die Embolisierung von Gefäßmißbildungen oder Gefäßen, die Tumoren versorgen. Auch hier wird das gesamte Verfahren durch die häufig wiederholte Injektion von Kontrastmitteln überwacht.

Im Gegensatz zu der normalen Röntgentechnik liefert die Computertomographie Schnittbilder durch den Körper, die eine sehr gute räumliche Auflösung erreichen. Obwohl auch die Dichteauflösung der Computertomographie deutlich höher ist als die Dichteauflösung der konventionellen Projektionsradiographie, werden zur sicheren Erkennung vieler krankhafter Veränderungen doch Kontrastmittel benötigt. Es hat sich als besonderer Vorteil erwiesen, daß neuere Geräte für eine einzelne Aufnahme (Schicht) nur noch weniger als eine Sekunde benötigen. Zur Charakterisierung pathologischer Veränderungen steht seither nicht nur der Kontrastmittelgehalt einer Läsion zu einem gegebenen Zeitpunkt zur Verfügung. Man kann nach rascher intravenöser Injektion das An- und Abfluten des Kontrastmittels exakt messen und daraus wesentliche zusätzliche Informationen, zum Beispiel über die Blutversorgung und Struktur eines Tumors erhalten. Da man in der Regel nur eine Schicht nach jeder einzelnen Kontrastmittelinjektion beobachten kann, sind wiederholte Injektionen notwendig.

Auch bei der Darstellung der Harnwege hat sich gezeigt, daß die schnelle Injektion einer hohen Kontrastmitteldosis zu den besten Ergebnissen führt.

Bedingt durch diese Entwicklungen sind die Anforderungen an die Qualität der Röntgenkontrastmittel ständig gestiegen.

1.) Die Konzentration

Die Röntgendichte eines Kontrastmittels ist von der Jodkonzentration in der verwendeten Lösung als einzigem Parameter abhängig, solange das Kontrastmittel nicht verdünnt wird. Das ist insbesondere in der Angiographie der Fall, wenn die Kontrastmittel mit hoher Geschwindigkeit über Katheter in Blutgefäße injiziert werden: Das Kontrastmittel verdrängt das Blut. Es ist umso besser geeignet, feinste Blutgefäße darzustellen, je höher die Jodkonzentration ist. Kontrastmittel mit extrem hoher Jodkonzentration sind insbesondere von Nutzen, wenn es darum geht festzustellen, ob ein Blutgefäß völlig verschlossen ist oder noch ein feiner Kanal als Verbindung zu intakten peripheren Gefäßen besteht und/oder ob periphere Gewebe durch feine Umgehungswege (Kollateralen) mit Blut versorgt werden können. Weiterhin kommt einer hohen Jodkonzentration große Bedeutung zu, wenn die Aufnahmebedingungen ansonsten ungünstig sind, zum Beispiel dadurch, daß der Strahlengang durch den Körper eines schweren Patienten sehr lang werden kann.

Bei einer Reihe von anderen Untersuchungen sind hochkonzentrierte Kontrastmittel ebenfalls erwünscht, entweder weil die Verdünnung im Körper sonst zu stark wird (Injektion in die Herzkammern, die Aorta oder bei der Intravenösen Digitalen Subtraktionsangiographie) oder weil einfach aus praktischen Gründen das Injektionsvolumen zur Erzielung einer bestimmten Dosis (g Jod/Patient oder g Jod/kg Körpergewicht) möglichst gering gehalten werden soll. Die bisher verfügbaren Röntgenkontrastmittel erreichen maximal eine praktisch nutzbare Jodkonzentration von 320 - 370 mg/ml. Die Ursache dafür ist, daß sie bei höherer Konzentration zu viskös und/oder zu schlecht verträglich werden. Verträglichkeitsprobleme entstehen vor allem durch die hohe Osmolalität der Lösungen bei hoher Jodkonzentration.

2.) Die Viskosität

Röntgenkontrastmittel sind in der Regel hochkonzentrierte Lösungen (50 bis >80 Gewichtsprozent). Diese Lösungen können sehr dickflüssig werden, zum Teil einfach deshalb, weil der Wassergehalt deutlich unter 1 g je ml Lösung liegt. Andererseits erfordert die Anwendung der Kontrastmittel häufig die sehr rasche Injektion großer Volumina (30 - 100 ml) durch möglichst nicht zu dicke Nadeln oder - noch problematischer - die Injektion durch bis zu über einen Meter lange Katheter, die zum Teil mehrere sehr enge Kanäle enthalten müssen, um außer der Kontrastmittelapplikation auch noch das Befüllen eines Ballons oder die Messung des lokalen Blutdrucks zu erlauben. Obwohl bei einigen Techniken bereits Druckinjektoren eingesetzt werden, muß die Viskosität der Kontrastmittellösungen doch auf ca. 10 cP bei 37 °C begrenzt werden.

Außer der schlechten Injizierbarkeit haben höher visköse Kontrastmittel auch den Nachteil schlechter Mischbarkeit mit Blut (Schlierenbildung statt homogener Füllung der Herzhöhlen oder Blutgefäße) und der Behinderung der Passage durch Kapillaren, zum Beispiel der Lunge (M. Langer, R. Felix, R. Keysser, U. Speck, D. Banzer: Beeinflussung der Abbildungsqualität der i.V. DSA durch die Jodkonzentration des Kontrastmittels. Digit. Bilddiagn. 5: 154-159 (1985). Aus all diesen Gründen sollten Röntgenkontrastmittel für die vasale Anwendung möglichst dünnflüssig sein.

3.) Die Osmolalität

Da Röntgenkontrastmittel für die meisten Anwendungen Jod in sehr hoher Konzentration enthalten müssen, waren die ursprünglich entwickelten Präparate gegenüber dem Blut und Gewebe sehr stark hyperton. Das hat eine Reihe von zum Teil schwerwiegenden Nebenwirkungen verursacht, so z.B. starke Schmerzen, Schädigungen der Blutgefäße und Herz-Kreislauf-Störungen. Zusätzlich wurde bei einigen Untersuchungen auch die Kontrastgebung durch starke osmotische Verdünnung der Kontrastmittel beeinträchtigt (Urographie).

Inzwischen sind durch neue, nichtionische Kontrastmittel Verbeserungen erzielt worden (B. Hagen: Iohexol and iopromide - Two new non-ionic water-soluble radiographic contrast media: Randomized, intraindividual double-blind study versus ioxaglate in peripheral angiography. In Contrast Media, Edited by Volker Taenzer and Eberhard Zeitler (1983), Georg Thieme Verlag, S. 104-114). Allerdings können in den schmerzhaften Indikationen nicht die kontrastreicheren, hochkonzentrierten Präparate (350 - 370 mg Jod/ml) eingesetzt werden, da diese mit >750 mosm/kg $H_2O$ noch immer die 2,5fache Osmolalität des Blutes aufweisen und damit zu schmerzhaft sind.

4.) Löslichkeit

Die wasserlöslichen Röntgenkontrastmittel leiten sich durchwegs vom Trijodbenzol ab. Diese Verbindung selbst ist praktisch unlöslich und recht toxisch. An dem Molekül verbleiben 3 Positionen, die mit Seitenketten zur Verbesserung der Löslichkeit und Verträglichkeit substituiert werden können. Beide Eigenschaften sind umso leichter zu optimieren je umfangreicher die Seitenketten werden. Große Substituenten bewirken aber gleichzeitig eine relative Abnahme des Jodgehalts im Molekül (in Prozent des Molekulargewichts), eine unerwünschte Zunahme der Viskosität sowie eine weitere Abnahme des ohnehin schon geringen Wassergehalts der hochkonzentrierten Lösungen, da bei gegebener Jodkonzentration jede Zunahme der Menge an organischer Substanz in der Lösung zu Lasten des Anteils an Wasser geht.

Es muß demnach ein Kompromiß zwischen möglichst hoher Wasserlöslichkeit und guter Verträglichkeit mit möglichst kleinen Seitenketten gefunden werden.

Die in der nachfolgenden Tabelle als 2) - 6) aufgeführten Verbindungen gelten als die zur Zeit bestgeeigneten Kontrastmittel. Man erkennt, das Molekulargewichte von 777 bis 821 bei einem Jodgehalt von 46 - 49 %, eine Viskosität von 8,1 cP (350 mg J/ml, 37 °C), eine Osmolalität von 680 mosm/kg $H_2O$ (370 mg J/ml) und ein $LD_{50}$-Wert (Maus) von 15 g Jod/kg als die im günstigsten Falle erzielbaren Werte anzusehen sind, wobei zu beachten ist, daß keine Verbindung diese derzeit optimalen Werte gleichzeitig aufweist.

TABELLE

**Eigenschaften von Kontrastmitteln für die Angiographie, Urographie und Computertomographie**

| | Molekular-gewicht | Jodgeh. % | Viskosität cP | Osmolalität mosm/kg $H_2O$ | $LD_{50}$ Maus i.v. g I/kg |
|---|---|---|---|---|---|
| | | | (37°C, 370 bzw. 350*mg I/ml) | | |
| 1) Beispiel 1 dieser Erfindung | 747 | 51 | 6,6 | 590 | 18 |
| 2) Iopromid | 791 | 48 | 9,5 | 770 | 15 |
| 3) Iopamidol | 777 | 49 | 9,4 | 830 | 15 |
| 4) Iohexol | 821 | 46 | 10,5 * | 820 * | 15 |
| 5) Iomeprol | 777 | 49 | 8,6 | 680 | 14 |
| 6) Ioxilan | 791 | 48 | 8,1 * | 700 * | 15 |

Inzwischen ist nun jedoch eine weitere Steigerung der Anforderungen zu registrieren: Wiederholte Injektionen auch in stark vorgeschädigte Gefäßgebiete, das Weglassen der früher gebräuchlichen Prämedikation mit Analgetika, der Verzicht auf den Einsatz von Narkosen bzw. auf das Zusetzen von Lokalanästhetika zu der Kontrastmittellösung, die insgesamt höheren Dosierungen und die z.T. wiederholte Untersuchung und/oder Behandlung von älteren und sehr kranken Patienten haben den Wunsch nach Kontrastmitteln mit noch niedrigerer Osmolalität und damit weiter verbesserter Verträglichkeit verstärkt. Für die bei ein und demselben Patienten oft wiederholt notwendige Aufweitung einzelner Abschnitte der Arterien sind solche besser verträglichen Kontrastmittel überhaupt eine Voraussetzung, um die Zustimmung der betreffenden Patienten für diese Art der Therapie zu erlangen. Da außerdem (s. oben) wesentliche praktische Eigenschaften der fertigen Kontrastmittellösung wie deren Injizierbarkeit durch feinste Katheter und das Fließ- und Kontrastverhalten in den Blutgefäßen direkt von der Viskosität abhängen, ist es wünschenswert, auch über Kontrastmittel zu verfügen, deren Viskositätsdaten im Vergleich zu den vorbekannten Mitteln erniedrigt sind.

Es besteht daher ein Bedarf an Röntgenkontrastmitteln, die diesen gesteigerten Anforderungen gerecht werden, wobei es darauf ankommt, nicht eine einzelne Eigenschaft zu Lasten anderer wichtiger Größen zu verbessern, sondern Substanzen zu finden, die möglichst vielen Anforderungen gerecht werden.

Diese Aufgabe wird durch die vorliegende Erfindung gelöst.

In der Europäischen Patentanmeldung mit der Publikationsnummer 0 015 867 sind Verbindungen beschrieben, die unter die beanspruchte allgemeine Formel I fallen :

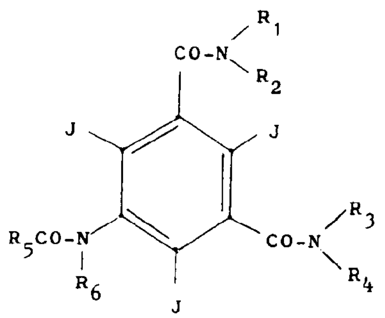

(I),

worin

die Amidreste $-CO-N.R_1R_2$ und $-CO-N.R_3R_4$ voneinander verschieden sind und

- $R_1$ ein Wasserstoffatom oder einen niederen Alkylrest,
- $R_2$ einen gerad- oder verzweigtkettigen Mono- oder Polyhydroxyalkylrest,
- $R_3$ ein Wasserstoffatom oder einen niederen Alkylrest,
- $R_4$ einen gerad- oder verzweigtkettigen Mono- oder Polyhydroxyalkylrest,
- $R_5$ niedere Alkyl- oder eine niedere Hydroxyalkyl- oder eine Niedere-alkoxy-niedere-alkylgruppe, und
- $R_6$ ein Wasserstoffatom oder einen gegebenenfalls hydroxylierten niederen Alkylrest

bedeuten.

Als bestgeeignete Verbindung wurde dabei Iopromid (Beispiele 6, 7, 8), das als Ultravist(R) auf dem Markt ist, erkannt (s. Tabelle).

Es wurde nun gefunden, daß überraschenderweise 5-Hydroxyacetamido-2,4,6-trijod-isophthalsäure-(2,3-dihydroxy-N-methylpropyl)-(2-hydroxyethyl)-diamid die vorstehend genannten Bedingungen erfüllt.

Diese Verbindung ist in der EP 0 015 867 nicht herausgestellt worden.

Es zeigte sich, daß die neue Verbindung trotz eines um 5 - 2 % gesteigerten Jodgehalts überraschend gut wasserlöslich ist. Die erfindungsgemäße Verteilung der hydrophilen Substituenten um den trijodierten Aromaten führt im Gegensatz zu den früher beschriebenen Verbindungen zu einer in vieler Hinsicht besseren Verträglichkeit.

Wie aus der Tabelle hervorgeht, hat die erfindungsgemäße Verbindung Nr.1 die jeweils günstigsten Werte aller sechs Kontrastmittel in sich vereinigt. Die Viskositäts- und Osmolalitäts-Werte gegenüber der besten in EP 0 015 867 genannten Verbindung (Iopromid) wurden um 30,5 bzw. 23,4 % verbessert; die größten Verbesserungen innerhalb der vorbekannten fünf Kontrastmittel betragen dagegen nur 22,9 % (Iohexol/Ioxilan) bzw. 18,1 % (Iopamidol/Iomeprol) und sind auch noch auf zwei verschiedene Verbindungen verteilt.

Die $LD_{50}$-Werte (Maus) sind die günstigsten der Tabelle. Aufgrund der herausragenden Eigenschaften der erfindungsgemäßen Verbindung ist es möglich, ohne die Blutgefäße zu schädigen, auch unter ungünstigsten Verhältnissen sehr feine oder wenig durchblutete Gefäße sichtbar zu machen. Es kommt zu einer vergleichsweise besonders geringen Beeinträchtigung der Blutbestandteile und Zellmembranen. Die gute Verträglichkeit des neuen Röntgenkontrastmittels erlaubt dessen Einsatz nicht nur in höherer Konzentration als bisher, sondern auch in sehr hoher Dosierung, bei sehr empfindlichen und vorgeschädigten Gefäßgebieten oder Patienten mit zahlreichen Erkrankungen einschließlich solcher des Herz-Kreislauf-Systems. Das neue Kontrastmittel ist wegen der raschen Injizierbarkeit, der glatten Passage durch die Lunge und der sehr schnellen, selektiv renalen Ausscheidung auch besonders gut für die Computertomographie, die Intravenöse Digitale Subtraktionsangiographie sowie die Urographie geeignet.

Schließlich ist zu berücksichtigen, daß die heute bevorzugten nichtionischen Röntgenkontrastmittel aufwendig in der Herstellung sind. Bedingt durch die sehr hohen Mengen je Patient (bis >200 g) sind die Kontrastmittel ein wesentlicher Kostenfaktor zumindest eines Teils der radiologischen Untersuchungen. Es ist daher ein Vorteil des beschriebenen Kontrastmittels, daß die günstigeren Eigenschaften erzielt wurden, ohne die Herstellungskosten weiter zu erhöhen.

Die pharmazeutische Zubereitung kann im allgemeinen beliebig den spezifischen Bedürfnissen des Verwenders angepaßt werden. Für die intravasale Injektion oder Infusion sind wäßrige Lösungen mit einem Kontrastmittelgehalt entsprechend 50 mg Jod/ml bis 450 mg Jod/ml, bevorzugt 100 bis 420 mg Jod/ml, geeignet. Diese Lösungen können physiologisch verträgliche Puffer (Bikarbonat, Phosphat, Citrat, Tris etc.), Stabilisatoren (EDTA, DTPA etc.), Elektrolyte ($Na^+$, $Ca^{2+}$, $K^+$, $Mg^{2+}$, $HCO_3^-$, $Cl^-$ etc.), Substanzen zur Anpassung der Osmolalität (Mannit, Glucose etc.) oder auch pharmakologisch wirksame Substanzen (Gefäßdilatatoren, Gerinnungshemmer etc.) enthalten. Dieselben Lösungen können auch zur Darstellung von beliebigen Körperhöhlen, Geweben oder sonstigen Strukturen verwandt werden, indem sie direkt in den darzustellenden Bereich injiziert oder sonstwie eingebracht werden.

Eine weitere Anwendung des betreffenden Kontrastmittels kann die orale Einnahme zur Darstellung des Magen-Darm-Trakts sein. Für diesen Zweck kann das Kontrastmittel als Pulver zur Herstellung einer Lösung vor Gebrauch oder als Konzentrat oder als fertige Lösung angeboten werden. In jedem Falle kann das Kontrastmittel physiologisch verträgliche Puffer, Stabilisatoren, Substanzen zur Anpassung der Osmolalität, pharmakologisch wirksame Substanzen, Konservierungsmittel, Geschmacksstoffe und/oder Quellstoffe enthalten.

Im allgemeinen wird das erfindungsgemäße Mittel in Mengen von 2 - 500, am häufigsten 20 - 200 ml/Untersuchung, dosiert.

Durch Zusatz des Kontrastmittel zu verschiedenen, in der Medizin benutzten Geräten, Präparaten oder Pharmaka kann deren Verbleib im Körper radiologisch kontrolliert werden.

Schließlich ist das neue Mittel wegen seines hohen spezifischen Gewichts, seiner geringen Viskosität und seiner physiologischen Eigenschaften auch als Dichtegradient-Medium in der isopycnischen Zentrifugation für die Fraktionierung biologischen Materials geeignet (Biological Separations in Iodinated Density Gradient Media, Information Retrieval Ltd., London and Washington D.C., Ed. D. Rickwood).

Die Erfindung betrifft ferner ein Verfahren zur Herstellung von 5-Hydroxyacetamido-2,4,6-trijod-isophthalsäure-(2,3-dihydroxy-N-methylpropyl)-(2-hydroxy-ethyl)-diamid, dadurch gekennzeichnet, daß man in an sich bekannter Weise

eine Verbindung der allgemeinen Formel II

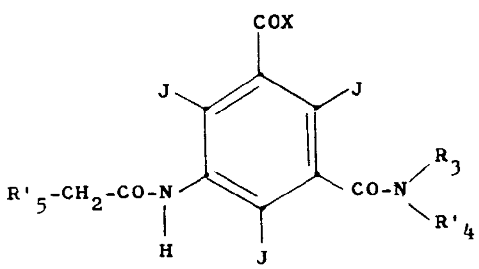

(II),

worin

$R_3$ ein Wasserstoffatom und

$R'_4$ eine 2-Hydroxyethylgruppe bzw.

$R_3$ eine Methyl- und $R'_4$ eine 2,3-Dihydroxypropylgruppe, wobei die Hydroxygruppe(n) gegebenenfalls geschützt ist/sind,

$R'_5$ eine geschützte Hydroxygruppe,

X ein reaktiver Säure- oder Esterrest

bedeuten,

mit einer Base der allgemeinen Formel III

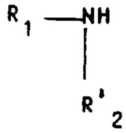

(III),

worin, wenn $R_3$ für ein Wasserstoffatom und $R'_4$ für eine gegebenenfalls geschütze 2-Hydroxyethylgruppe steht,

$R_1$ eine Methylgruppe und

$R'_2$ eine 2,3-Dihydroxypropylgruppe, deren Hydroxygruppen gegebenenfalls geschützt sind,
bzw. wenn $R_3$ für eine Methylgruppe und $R'_4$ für eine gegebenenfalls geschützte 2,3-Dihydroxygruppe steht,
$R_1$ ein Wasserstoffatom und
$R'_2$ eine 2-Hydroxyethylgruppe, deren Hydroxygruppe gegebenenfalls geschützt ist,
bedeuten, umsetzt und anschließend die vorhandene(n) Hydroxyschutzgruppe(n) abspaltet.

Als reaktiver Säure- oder Ester-Rest X kommt insbesondere ein Halogen, wie -Cl, -Br oder -J infrage. Grundsätzlich durchführbar ist die Umwandlung aber auch, wenn X den Azidrest, einen Alkoxycarbonyloxyrest oder den Rest einer reaktiven Estergruppe, z.B. einen üblichen -O-Alkyl, O-Aryl oder $-O-CH_2-C\equiv N$ bedeutet; bevorzugt geht sie von Ausgangsprodukten mit X in der Bedeutung Cl aus.

Die in den Ausgangssubstanzen II und III anwesenden Hydroxylgruppen können in freier oder geschützter Form vorliegen. Sollen diese Hydroxylgruppen in geschützter Form vorliegen, kommen alle Hydroxylschutzgruppen infrage, die bekanntermaßen für einen intermediären Hydroxylgruppenschutz geeignet sind, d.h. die sich leicht einführen und sich später unter Rückbildung der letztlich gewünschten freien Hydroxylgruppe auch wieder leicht abspalten lassen. Bevorzugt ist der Schutz durch Veresterung, z.B. durch Einführung des Benzoyl-, Alkanoyl- oder Acyl- insbesondere des Acetylrestes, und im Falle von 1,2-Diolen auch des cyclischen Sulfitesters [Topics in Stereochemistry, Vol. 13 (1982), 364]. Geeignete Schutzgruppen sind auch Äthergruppen wie z.B. Benzyl-, Di- und Triphenylmethyl-Äthergruppen sowie Acetal- und Ketalgruppen mit z.B. Acetaldehyd, Aceton oder Dihydropyran.

Die Amidierungsreaktion erfolgt in einem geeigneten Lösungsmittel bei 0 - 100 °C, vorzugsweise bei 20 - 80 °C. Geeignete Lösungsmittel sind u.a. polare Lösungsmittel. Beispielsweise genannt seien Aceton, Wasser, Dioxan, Ethylenglykoldimethylether, Tetrahydrofuran, Dimethylformamid, Dimethylacetamid, Hexametapol u.ä. und deren Gemische. Da die Amidierungsreaktion exotherm verläuft, ist es gegebenenfalls zweckmäßig, das Reaktionsgemisch leicht zu kühlen, um die Reaktionstemperatur auf etwa 50 °C halten zu können. Da mit der Amidierungsreaktion HX frei wird, benötigt man zwecks Neutralisation pro COX-Gruppe zwei Äquivalente Base, zweckmäßigerweise im Überschuß von ca. 10 %. Zur praktischen Durchführung wird das gelöste oder suspendierte Ausgangsprodukt II umgesetzt mit 2 Äquivalenten der Base III oder mit einem Äquivalent der Base III und einem Äquivalent einer von III verschiedenen Base, die dann als Protonenakzeptor dient.

Als Protonenakzeptoren zur Neutralisation verwendet man vorteilhaft tertiäre Amine, wie z.B. Triäthylamin, Tributylamin oder Pyridin, sowie anorganische Basen wie Alkali- oder Erdalkalihydroxide, -carbonate oder -hydrogencarbonate, z.B. Natriumhydroxid, Natriumhydrogencarbonat, Kaliumcarbonat, Natriumcarbonat, Calciumhydroxid oder Magnesiumhydroxid.

Die im Reaktionsverlauf anfallenden anorganischen oder organischen Salze werden in bekannter Weise abgetrennt, vorteilhaft z.B. mit Hilfe üblicher Ionenaustauscher-Säulen oder durch Filtration über bekannte Adsorbentien wie z.B. Diaion oder Amberlite XAD-d2 und -4.

Die spätere Abspaltung der intermediär eingeführten Schutzgruppen unter Freisetzung der letztlich gewünschten Hydroxylgruppen erfolgt ebenfalls nach Methoden, die dem Fachmann allgemein geläufig sind. So kann die Abspaltung der Schutzgruppen ohne besondere Reaktionsstufe mit der Aufarbeitung und Isolierung der Umsetzungsprodukte erfolgen. Sie kann aber auch in üblicher Weise in einer getrennten Reaktionsstufe durchgeführt werden. Acylschutzgruppen können beispielsweise durch alkalische und Acetal-, Ketal- oder Ätherschutzgruppen durch saure Hydrolyse abgespalten werden.

Das Ausgangsprodukt der Formel II erhält man zweckmäßigerweise aus dem leicht zugänglichen 5-Nitroisophthalsäure-mono-ethylester. Durch Aminolyse der Estergruppe wird zunächst der Amidrest $-N.R_3R'_4$ eingeführt. Liegen anwesende Hydroxylgruppen im Amidrest in freier Form vor, werden diese gegebenenfalls in üblicher Weise, z.B. als O-Acetat, geschützt. Die anschließende Reduktion der Nitrogruppe zur aromatischen Aminogruppe erfolgt ebenfalls nach an sich bekannten Methoden z.B. mit Raney-Nickel oder Pd auf $CaCO_3$ in Gegenwart von Wasser oder eines niederen Alkohols wie Methanol oder Äthanol bei normalem oder erhöhtem Druck. Das so erhaltene 5-Amino-isophthalsäure-monoamid wird nun in üblicher Weise trijodiert und die freie Carboxylgruppe in die Säurehalogenidgruppe, vorzugsweise in die -COCl-Gruppe, überführt. Wird in die Umsetzung mit Thionylchlorid ein 2,3-Dihydroxypropylamid eingesetzt, so werden (wie oben erwähnt) die vicinalen Diolgruppen gleichzeitig als Sulfitester geschützt. Man kann aber auch diese Hydroxylgruppen zunächst z.B. als Acetate schützen und anschließend die Carboxylgruppe in das Säurehalogenid überführen. Anschließend wird die aromatische Aminogruppe in üblicher Weise mit einem reaktiven $R'_5-CH_2-CO$-Säurederivat zum Ausgangsprodukt der allgemeinen Formel II N-acyliert, indem man z.B. das Amin in einem inerten Lösungsmittel, wie z.B. Essigester, Ethylenglykoldimethylether, Dioxan, THF, Dichlorethan, Pyridin, DMA, DMF u.ä. bei Temperaturen von 0 °C bis 100 °C mit einem reaktiven $R'_5-CH_2-CO$-Säurederivat, vorzugsweise mit dem entsprechenden Säurehalogenid, insbesondere Säurechlorid, oder aber auch mit einem entsprechenden Säureanhydrid, vorzugsweise in Gegenwart eines sauren Katalysators, wie z.B. $H_2SO_4$, umsetzt. Die Einführung des $R'_5$-$CH_2$-CO-Restes kann jedoch auch vor der Bildung des Säurehalogenids vorgenommen werden.

**Beispiel 1**

5-Hydroxyacetamido-2,4,6-trijod-isophthalsäure-(2,3-dihydroxy-N-methylpropyl)-(2-hydroxy-ethyl)-diamid

a) **5-Amino-2,4,6-trijod-isophthalsäure-(2-acetoxy-ethyl)-monoamid**

300,96 g (500 mmol) 5-Amino-2,4,6-trijod-isophthalsäure-(2-hydroxyethyl)-monoamid (GB 1 146 133) werden in 300 ml Dioxan suspendiert, 6,1 g (50 mmol) 4-Dimethylamino-pyridin und 56,4 ml (600 mmol) Acetanhydrid hinzugefügt und das Reaktionsgemisch bei einer Innentemperatur von 80 °C gerührt. Nach ca. 1 Stunde liegt eine Lösung vor, nach 2 Stunden ist die Umsetzung quantitativ. Die Reaktionslösung wird auf Raumtemperatur gekühlt, mit 300 ml Essigester verdünnt, mit authentischen Kristallen geimpft und das Produkt innerhalb von 5 Stunden auskristallisiert. Es wird abgesaugt, mit Essigester gewaschen und bei 50 °C im Vakuum getrocknet. Man erhält 321 g (442,3 mmol) = 88,46 % d.Th. unter Berücksichtigung eines Solvatgehalts von 10,2 % Dioxan.

Analyse unter Berücksichtigung von 10,2 % Dioxan, 0,25 % Essigester und 0,82 % Wasser:

| | | | | | | |
|---|---|---|---|---|---|---|
| berechnet: | C 25,55 | H 2,57 | I 52,45 | N 3,86 | O 15,54 | % |
| gefunden: | 25,83 | 2,68 | 52,21 | 4,05 | | % |

b) **5-Amino-2,4,6-trijod-isophthalsäure-(2-acetoxy-ethyl)-monoamid-chlorid**

577 g (800 mmol unter Berücksichtigung eines Gehalts von 12 % Dioxan) 5-Amino-2,4,6-trijod-isophthalsäure-(2-acetoxy-ethyl)-monoamid werden in 2,88 l Dichlorethan suspendiert, 174,2 ml (2,4 mol) Thionylchlorid hinzugefügt und das Reaktionsgemisch bei einer Badtemperatur von 120 °C am Rückfluß gekocht. Nach 1 Stunde ist aus der Suspension eine Lösung entstanden. Nach 3 Stunden ist die Umsetzung vollständig. Unter vermindertem Druck werden das überschüssige Thionylchlorid und das Dichlorethan weitgehend abdestilliert. Der teils ölige, teils feste Rückstand wird in 1,3 l Dichlorethan aufgenommen und portionsweise 228,9 g (800 mmol) Sodadekahydrat zugesetzt, um das Sulfinylimid zu hydrolysieren. Die Farbe der Suspension wechselt von Orange nach Hellgelb. Es wird filtriert, der Filterrückstand mit 1,8 l Tetrahydrofuran heiß extrahiert, filtriert, das THF-Filtrat eingedampft und bei 50 °C im Vakuum getrocknet. Man erhält 487,1 g (735,4 mmol) des Säurechlorids.

Analyse:

| | | | | | | | |
|---|---|---|---|---|---|---|---|
| berechnet: | C 21,76 | H 1,52 | Cl 5,35 | I 57,48 | N 4,23 | O 9,66 | % |
| gefunden: | 22,15 | 2,03 | 5,67 | 56,98 | 4,03 | | % |

c) **5-Acetoxyacetamido-2,4,6-trijod-isophthalsäure-(2-acetoxy-ethyl)-amid-chlorid**

238,5 g (360 mmol) 5-Amino-2,4,6-trijod-isophthalsäure-(2-acetoxy-ethyl)-monoamid-chlorid werden in 1,19 l Dioxan bei Raumtemperatur gelöst. Man fügt 146,5 g (1,08 mol) Acetoxyacetylchlorid hinzu und rührt die Reaktionslösung bei 80 bis 90 °C. Nach 12 Stunden ist die Umsetzung praktisch quantitativ. Es wird auf Raumtemperatur gekühlt, angeimpft und 10 Stunden kristallisiert. Das Kristallisat wird abgesaugt, mit Dioxan gewaschen und bei 50 °C im Vakuum getrocknet. Man erhält 246,5 g (289,67 mmol unter Berücksichtigung von 10,4 % Dioxan als Solvat) = 80,5 % d.Th. kristallines Produkt.

Analyse unter Berücksichtigung von 10,4 % Dioxan als Solvat:

| | | | | | | | |
|---|---|---|---|---|---|---|---|
| berechnet: | C 28,23 | H 2,61 | Cl 4,16 | I 44,69 | N 3,28 | O 16,99 | % |
| gefunden: | 28,18 | 2,44 | 4,18 | 44,96 | 3,35 | | % |

d) **5-Hydroxyacetamido-2,4,6-trijod-isophthalsäure-(2,3-dihydroxy-N-methyl-propyl)-(2-hydroxy-ethyl)-diamid**

Bei Raumtemperatur werden 533,7 g (0,70 mol) 5-Acetoxyacetamido-2,4,6-trijod-isophthalsäure-(2-acetoxy-ethyl)-amid-chlorid und 300,4 g (1,05 mol) Natriumcarbonat-Dekahydrat in 2,67 l Aceton suspendiert und 95,7 g (0,91 mol) N-Methylamino-propandiol-2,3 zugetropft. Nach beendeter Zugabe des Amins wird die Suspension 1 Stunde am Rückfluß gekocht. Es wird dann auf Raumtemperatur gekühlt, der Feststoff abgesaugt und das Filtrat weitgehend eingedampft. Der ölige Rückstand wird 1:1 in Wasser gelöst, die Lösung auf 50 °C erwärmt und durch kontinuierliche Zugabe von 32 %iger NaOH bei pH 11 - 11,5 verseift. Nach beendeter Hydrolyse der Acetoxygruppen wird mit HCl neutralisiert und die Lösung an je 3-Liter-Kat- und Anionenaustauscher entsalzt. Das wäßrige Eluat wird unter vermindertem Druck zu einem Schaum eingedampft. Man erhält 450 g amorphes Rohprodukt. Dieses wird in 750 ml Ethanol in der Siedehitze gelöst, mit authentischen Kristallen geimpft und 12 Stunden bei 60 °C kristallisiert. Das Kristallisat wird abgesaugt, mit Ethanol gewaschen und 32 Stunden bei 50 °C im Vakuum getrocknet. Man erhält 345,4 g (462,35 mmol) = 66,95 % d.Th. Kristallisat. Fp. 258 - 260 °C.

Analyse unter Berücksichtigung von 0,39 % Wasser und 0,1 % Ethanol:

| | | | | | | |
|---|---|---|---|---|---|---|
| berechnet: | C 25,65 | H 2,74 | I 50,71 | N 5,59 | O 15,29 | % |
| gefunden: | 25,73 | 2,79 | 50,67 | 5,58 | | % |

<u>Alternative Herstellung von 5-Hydroxyacetamido-2,4,6-triiod-isophthalsäure-(2,3-dihydroxy-N-methyl-propyl)-(2-hydroxy-ethyl)-diamid</u>

74,85 g (100 mmol) 5-Amino-2,4,6-triiod-isophthalsäure-(2,3-diacetoxy-N-methyl-propyl)-amid-chlorid [Beispiel 8c) in der DOS 29 09 439] werden in 225 ml trockenem Essigester gelöst, 34,13 g (250 mmol) Acetoxyacetylchlorid zugefügt und 5 Stunden am Rückfluß gekocht. Die Reaktionslösung wird dann unter vermindertem Druck zu einem Öl eingedampft, dieses Öl in 225 ml Aceton gelöst, mit 57,23 g (200 mmol) Sodadekahydrat und 9,16 g (150 mmol) Ethanolamin versetzt und 5 Stunden bei Raumtemperatur gerührt. Die Suspension wird dann filtriert und das Filtrat zu einem Öl eingedampft. Das Öl wird in 300 ml Wasser gelöst, die Lösung auf 50 °C erwärmt und durch portionsweise Zugabe von konzentrierter Natronlauge bei pH 11 - 11.5 die O-Acetat-Gruppen verseift. Nach beendeter Hydrolyse wird mit Salzsäure neutralisiert und die Lösung an je 1 Liter Kationen- und Anionenaustauscher entsalzt. Das wäßrige Eluat wird unter vermindertem Druck zu einem Schaum eingedampft. Man erhält 65 g amorphes Produkt. Dieses wird in 100 ml siedendem Ethanol gelöst, die Lösung in der Siedehitze mit authentischen Kristallen geimpft und 12 Stunden bei 60 - 70 °C kristallisiert. Das Kristallisat wird abgesaugt, mit Ethanol gewaschen und 48 Stunden bei 50 °C im Vakuum getrocknet. Man erhält 51,02 g (68,3 mmol) = 68,3 % d. Th. bezogen auf das eingesetzte Amin.

**Beispiel 2**

1,5 l einer Injektionslösung mit 370 mg I/ml der Verbindung aus Beispiel 1d) werden hergestellt, indem man 1089,1 g der Jodverbindung in 500 ml bidestilliertem Wasser auflöst, die Lösung mit 1,89 g Natriumbicarbonat auf pH 7,3 einstellt, 162,3 mg $CaNa_2EDTA$ hinzufügt, auf ein Volumen von 1 Liter mit bidestilliertem Wasser auffüllt, durch ein Filter der Porenweite 0,22 µm filtriert, in Multivials abfüllt und 20 Minuten bei 120 °C sterilisiert.

**Beispiel 3**

1 l einer Injektionslösung mit 370 mg I/ml der Verbindung aus Beispiel 1d) werden hergestellt, indem man 726,06 g der Iodverbindung in 330 ml bidestilliertem Wasser löst, 1,211 g α,α,α-Tris-(hydroxymethyl)-methylamin und 108,2 mg $CaNa_2EDTA$ hinzufügt, mit 5,6 ml 1N Salzsäure auf pH 7,3 einstellt, durch ein Filter der Porenweite 0,22 μm filtriert, in Multivials abfüllt und 20 Minuten bei 120 °C sterilisiert.

**Beispiel 4**

Zur oralen Anwendung werden 100 g der Verbindung aus Beispiel 1d) mit 5 g Saccharose, 0,5 g Aromastoff und 100 mg Polyoxyethylen-Polyoxypropylen-Polymer innig vermischt und steril verpackt. Dieses Pulver wird als Suspension in Wasser verabreicht.

## Patentansprüche

1. 5-Hydroxyacetamido-2,4,6-trijod-isophthalsäure-(2,3-dihydroxy-N-methylpropyl)-(2-hydroxy-ethyl)-diamid.

2. Verfahren zur Herstellung von 5-Hydroxyacetamido-2,4,6-trijod-isophthalsäure-(2,3-dihydroxy-N-methyl-propyl)-(2-hydroxy-ethyl)-diamid, dadurch gekennzeichnet, daß man in an sich bekannter Weise eine Verbindung der allgemeinen Formel II

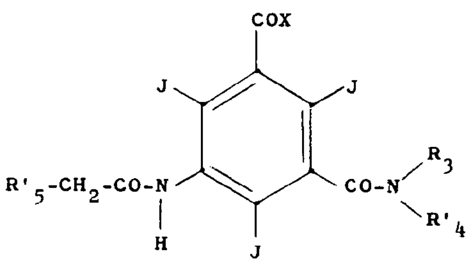

(II),

worin

$R_3$ ein Wasserstoffatom und

$R'_4$ eine 2-Hydroxyethylgruppe bzw.

$R_3$ eine Methyl- und $R'_4$ eine 2,3-Dihydroxypropylgruppe, wobei die Hydroxygruppe(n) gegebenenfalls geschützt ist/sind,

$R'_5$ eine geschützte Hydroxygruppe,

X ein reaktiver Säure- oder Esterrest

bedeuten,

mit einer Base der allgemeinen Formel III

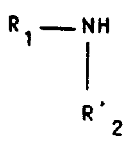

(III),

worin, wenn $R_3$ für ein Wasserstoffatom und $R'_4$ für eine gegebenenfalls geschützte 2-Hydroxyethylgruppe steht,

$R_1$ eine Methylgruppe und

$R'_2$ eine 2,3-Dihydroxypropylgruppe, deren Hydroxygruppen gegebenenfalls geschützt sind,

bzw. wenn $R_3$ für eine Methylgruppe und $R'_4$ für eine gegebenenfalls geschützte 2,3-Dihydroxygruppe steht,

$R_1$ ein Wasserstoffatom und

$R'_2$ eine 2-Hydroxyethylgruppe, deren Hydroxygruppe gegebenenfalls geschützt ist,

bedeuten, umsetzt und anschließend die vorhandene(n) Hydroxyschutzgruppe(n) abspaltet.

3. Röntgenkontrastmittel enthaltend die Verbindung gemäß Anspruch 1 als schattengebende Substanz.

4. Verwendung der Verbindung gemäß Anspruch 1 zur Herstellung von Röntgenkontrastmittel.

## Claims

1. 5-Hydroxyacetamido-2,4,6-triiodoisophthalic acid (2,3-dihydroxy-N-methylpropyl)-(2-hydroxyethyl)-diamide.

2. Process for the preparation of 5-hydroxyacetamido-2,4,6-triiodoisophthalic acid (2,3-dihydroxy-N-methylpropyl)-(2-hydroxyethyl)-diamide, characterised in that
a compound of the general formula II

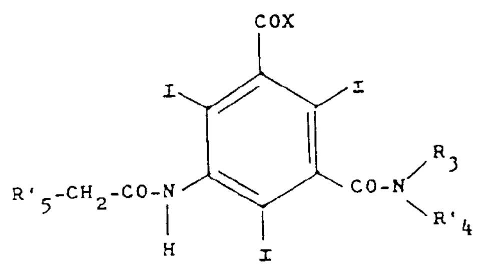

(II),

wherein
$R_3$ represents a hydrogen atom and
$R'_4$ represents a 2-hydroxyethyl group, or
$R_3$ represents a methyl group and $R'_4$ represents a 2,3-dihydroxypropyl group, the hydroxy groups(s) optionally being protected,
$R'_5$ represents a protected hydroxy group, and
X represents a reactive acid or ester radical,
is reacted in a manner known
per se with a base of the general formula III

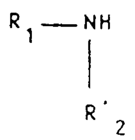

(III),

wherein, when $R_3$ represents a hydrogen atom and $R'_4$ represents an optionally protected 2-hydroxyethyl group,
$R_1$ represents a methyl group and
$R'_2$ represents a 2,3-dihydroxypropyl group, the hydroxy groups of which are optionally protected,
or, when $R_3$ represents a methyl group and $R'_4$ represents an optionally protected 2,3-dihydroxy group,
$R_1$ represents a hydrogen atom and
$R'_2$ represents a 2-hydroxyethyl group, the hydroxy group of which is optionally protected,
and then the hydroxy-protecting group(s) present is(are) removed.

3. X-ray contrast media containing the compound according to claim 1 as radio-opaque substance.

4. Use of the compound according to claim 1 in the preparation of X-ray contrast media.

## Revendications

1. (2,3-Dihydroxy-N-méthylpropyl)-(2-hydroxyéthyl)-diamide de l'acide 5-hydroxyacétamido-2,4,6-triiodoisophtalique.

2. Procédé de préparation du diamide de la revendication 1, procédé caractérisé en ce que l'on fait réagir d'une manière en elle-même connue un composé de formule générale II

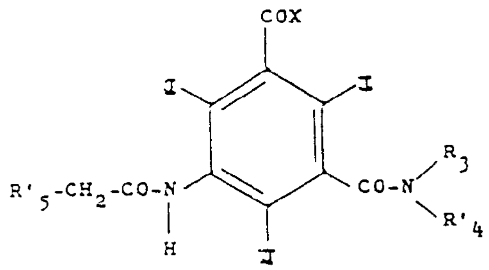

(II),

(dans laquelle

$R_3$ représente un atome d'hydrogène et

$R'_4$ un groupe 2-hydroxyéthyle ou bien

$R_3$ le groupe méthyle et $R'_4$ un groupe 2,3-dihydroxypropyle, le ou les groupes hydroxyliques étant éventuellement protégés,

$R'_5$ représente un groupe hydroxylique protégé, et

X un radical d'acide ou d'ester réactif)

avec une base de formule générale III

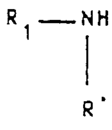

(III),

(dans laquelle, si $R_3$ est un atome d'hydrogène et $R'_4$ un groupe 2-hydroxyéthyle éventuellement protégé,

$R_1$ est un groupe méthyle et

$R'_2$ un groupe 2,3-dihydroxypropyle dont les hydroxyles sont éventuellement protégés,

ou bien si $R_3$ est le groupe méthyle et $R'_4$ un groupe 2,3-dihydroxypropyle éventuellement protégé,

$R_1$ est un atome d'hydrogène et

$R'_2$ est un groupe 2-hydroxyle dont l'hydroxyle est éventuellement protégé),

puis on élimine le ou les groupes protecteurs éventuels des groupes hydroxyliques.

3. Agent de contraste pour rayons X comprenant le composé de la revendication 1 pour donner le contraste.

4. Emploi du composé de la revendication 1 pour la préparation d'agents de contraste pour rayons X.